# EUROPEAN PATENT APPLICATION

(11) **EP 1 351 053 A1**
(43) Date of publication of application: **08.10.2003**
(21) Application number: 02290862.8
(22) Date of filing: 05.04.2002
(51) Int. Cl.: G01N 33/00, G01N 35/10

(54) **Method for preparing fragrance composition with plurality of sampling ports**

(71) Applicant: Quest International Fragrance Company, Mt. Olive, New Jersey 07828 (US)
(72) Inventor: Ansarih,Rahman, Old Tappan, New Jersey 07675 (US); de Chiris, Yves, Endoufielle (FR); Waran, Ravi, Hackettstown, New Jersey 07840 (US)
(74) Representative: Matthews, Heather Clare

(57) **Abstract**

The invention concerns a method for preparing a fragrance composition which comprises sampling from a plurality of sampling points, each containing a fragrance component, using a plurality of samplers, one sampler transferring an aliquot from one such sampling point to a mixing container, mixing the aliquots and testing the hcdonic effect against a fragrance concept.

## Description

This invention relates to a method for designing fragrances.

Fragrance design is a unique blend of artistry and scientific understanding. A successful fragrance much like fine art or music must be able to evoke mood and favourable emotion in the user and those who perceive it. Designing a successful fragrance therefore requires great creativity on the part of the Perfumer as it does from a painter or composer.

The design of fragrances usually comes about when a fashion house, a fashion designer, a branded fragrance house or a consumer products company gives a creative brief to a Perfumer. Such a brief may be either specific to the type of fragrance to be created in terms of its hedonic effect e.g. to be high in citrus or musk notes or it may be broadly defined in terms of mood, e.g. to be warm, sumptuous and sensual. Either way the expectation on the part of the person presenting the brief would be for the fragrance to be somehow new and creative in its sensory impact.

Classically, fragrance compositions are made up by a Perfumer and/or his technician by hand, sampling and weighing from a collection of standard fragrance components. A typical fragrance composition may contain from 50-250 different such components. The mixture so obtained, the fragrance composition, is then smelt by the Perfumer, the formula altered and the process repeated again and again until the hedonic effect is optimised. This overall process can often take days or even weeks.

In such a classical process rules have evolved (much like in painting where primary colours are mixed, e.g. yellow and blue to make green) which a Perfumer may use to make first approximations for the type of mood or hedonic effect that he wants to achieve. However, unlike painting, the creative process is very laborious and the rules themselves stifle creativity in that they dampen spontaneity.

In an unrelated art, that is pharmacological testing and screening, apparatus as been developed to carry out large numbers of routine tests in parallel either to check the activity of pharmaceutical formulations or for diagnostic purposes. Typically, such apparatus comprises a number of sampling points some of which contain materials under examination and others which contain standard reagents or exipients. Samplers transfer aliquots of the material under examination from a number of sampling points to a number of mixing points where that material is mixed with a standard reagent or with an exipient and the mixture is then subjected to a number of tests e.g. plating out on petri dishes where the biological activity can be observed.

We have now found that it is possible to modify such equipment for use in the design of fragrances where unexpectedly it unlocks the Perfumer's creativity becoming almost like a musical instrument in his hands enabling him to create symphonys of fragrance.

Accordingly, the present invention provides a method for preparing a fragrance composition which comprises sampling from a plurality of sampling points, each containing a fragrance component, using a plurality of samplers, one sampler transferring an aliquot from one such sampling point to a mixing container, mixing the aliquots and testing the hedonic effect against a fragrance concept.

A fragrance component is a pure substance having a fragrant effect which may be mixed with others to produce a fragrance composition. Examples of fragrance components are limettal, gardamousse, fleuroxene and 1-carvone. Such materials are known in the art and are described in standard works for example "Common Flavour and Fragrance Materials" by Kurt Bauer, Dorothea Garbe and Horst Surburge (third edition published in 2000), S. Arctander "Purfume and Flavour Chemicals (Montclair, N.J. 1969), S. Arctander "Purfume and Flavour Materials of Natural Origin (Elizabeth, N.J., 1960) and in "Flavour and Fragrance Materials - 1991" (Allurel Publishing Co. Wheaton, III.USA).

Apparatus which can be adapted for this method of design is known. One such machine that is used in pharmacological testing is the Genesis Robotic Sample Processor. This is available commercially from Tecan Schweiz AG.

Such apparatus adapted specifically for use in this method comprises at least 10 but more usually at least 16 sampling points each of which contain a fragrance component. In practice it will not generally contain more than 3,000 sampling points.

A working collection (or organ of fragrance) typically comprises 200 to 250 components and a work bench of fragrance comprises up to 1,500 such components. Hence apparatus employed with such collections of fragrances will include a corresponding number of sampling points, one for each fragrance component. So the apparatus includes at least 200 to 250 sampling points. More typically it will include from 500 to 2,000 sampling points and preferably from 1,000 to 2,000 sampling points.

The apparatus will also comprise of at least 4 samplers. The sampler is a device which can withdraw a measured amount of fragrance component from a sampling point and transfer it to the mixing container. It can be for example a pipette or a syringe. In this art the pipette sampler is referred colloquially as a "tip".

The apparatus in practice will contain not more than 20 such samplers. For example it will contain from 4-12 samplers. Generally it will contain about 6-8 samplers.

The apparatus also includes a cleaning arrangement where each sampler can be cleaned after it has transferred an aliquot from the sampling point to the container before taking an aliquot from a second sampling point so that the second sampling point is not contaminated. In use, the apparatus is so arranged that one or more samplers can each be collecting an aliquot whilst one or more others can be cleaned for example with washing fluid in particular ethanol.

The sampling preferably occurs rapidly since that assists the Perfumer's creative flow. For example each sampler should be able to sample at a rate of at least 5 aliquots per minute. Generally a sampling rate will not be greater than 15 aliquots per minute for example 8 or 10 aliquots per minute.

Preferably the aliquots are small for example less that 10µl or microlitres. Generally the aliquots are from 1- 300µl or microlitres inclusive. Typically they are from 5-250µl or microlitres, for example 5µl, 10µl or 15µl. Generally the total volume of prototype fragrance composition produced is 5ml or thereabouts.

Preferably the components are dissolved in a non-interfering solvent, one that is compatible with the fragrance components in that it does not react to any appreciable extent with any of the fragrance components under investigation by this method. Such solvents are known. An example is ethanol. The total concentration of fragrance components in such solutions is in the range of 1-50 percent by volume of components to solvent. Working with fragrance components in solution promotes ease of mixing since viscosity and specific gravity differences between components can be minimised. Such solutions are relatively easy to handle. As the concentrations of the solution will be known the amounts used by volume can be converted readily into gravimetric equivalents. This allows the weight ratio of the components of the final scaled up fragrance composition, based on and having the fragrance of the prototype fragrance composition, to be determined accurately.

Preferably the container for the mixture has means capable of vaporising the fragrance composition so that it can be assessed for smell immediately.

The surprising result of employing this method of design is that it has allowed fragrance samples to be produced in a rapid stream unlike standard systems in which they are produced in a step-like fashion. This in turn allows the Perfumer to work creatively and spontaneously with fragrance and to improvise much as a jazz musician can improvise in an extemporary performance.

Classic fragrance design operates on a combination of rules. Creativity calls for creative discontinuity: that is where components are put together in such a way that gives effects that established rules would not anticipate. This method of this invention not only allows these rules to be put aside to allow improvisation but it also increases the rate at which this creative discontinuity can occur.

The following examples illustrate the invention.

### Example 1

A 10ml sample of fragrance composition was made using the Genesis Robotic Sample Processor by sampling aliquots listed in Table 1 from the components listed in that Table.

**Table 1**

| | |
|---|---|
| Aldehyde C 8 (octanal) | 0.100 |
| Aldehyde C 9 (nonanal) | 0.100 |
| Aldehyde C11 (undecylenic) 10% DPG | 0.100 |
| Aldehyde MNA | 0.100 |
| Amylbutyrate 10% DPG AA 5575 | 0.400 |
| Amylbutyrate | 0.100 |
| Amylcinnamic aldehyde | 1.000 |
| Amylsalicylate | 5.000 |
| Benzylacetate extra | 10.000 |
| Cerdarwood Texan light | 2.400 |
| Cedarwood Virginian pure | 3.600 |
| Coumarin | 7.000 |
| Cyclamen aldehyde | 2.700 |
| Diethyl phthalate | 5.500 |
| Diphenyl oxide | 3.000 |
| Cis 1-hexenol 10%DEP AA 0216 | 0.200 |
| Eucalyptus 80/85 | 0.500 |
| Paracresyl phenylacetate | 0.100 |
| Phenyl ethyl alcohol | 33.000 |
| Pineamerican | 0.100 |
| Rosemary French | 0.400 |
| Spice Spanish | 0.200 |
| Terpineol | 17.300 |
| Terpinyl acetate | 5.000 |
| Turpentine steam distilled | 0.100 |
| Vanillin | 2.000 |
| | |
| | |
| (1000.00 quantity units = 10ml) | Ingredient quantities: 100.000 |

### Example 2

A 10ml fragrance composition was made using the Genesis Robotic Sample Processor by sampling aliquots listed in Table 2 from the components in that Table.

**Table 2**

| | |
|---|---|
| Amyris | 1.300 |
| Benzaldehyde 1% DEP AA 0074 | 0.150 |
| Benzyl acetate extra | 18.900 |
| Benzyl formate | 6.800 |
| Celestolide 1% DEP AA 6360 | 0.500 |
| Citronellol pure | 12.800 |
| Cortex aldehyde 50 50% DEP AA 6502 | 1.250 |
| Diethyl phthalate | 0.650 |
| Efetaal | 0.100 |
| Galaxolide solvent free | 0.200 |
| Geraniol pure | 11.700 |
| Geranyl acetate | 4.200 |
| Guaiacwood pure | 3.200 |
| Gurjun balsim light | 0.150 |
| Heliotropin 1% DPG AA 4219 | 0.100 |
| Hydratropic aldehyde | 1.700 |
| Ionone | 5.900 |
| Methyl naphthyl ketone 1 %DEP AA 4407 | 0.300 |
| Musk ketone reconstituted AB 7298 | 0.800 |
| Nerol 90 | 2.100 |
| Nutmeg pure | 0.800 |
| Phenyl ethyl alcohol | 24.200 |
| Phenyl ethyl acetate | 0.200 |
| Sandalwood | 1.000 |
| Tetrahydro geraniol | 1.000 |
| | |
| (100.00 quantity units = 10ml) | Ingredient quantities: 100.000 |

## Claims

1. A method for preparing a fragrance composition which comprises sampling from a plurality of sampling points, each containing a fragrance component, using a plurality of samplers, one sampler transferring an aliquot from one such sampling point to a mixing container, mixing the aliquots and testing the hedonic effect against a fragrance concept.

2. A method as claimed in Claim 1 in which there are at least 10 sampling points.

3. A method as claimed in Claim 1 or Claim 2 where there are up to 3,000 sampling points.

4. A method as claimed in any one of Claims 1 to 3 where there are at least 4 samplers.

5. A method as claimed in any one of Claims 1 to 4 where there are up to 20 samplers.

6. A method as claimed in any one of Claims 4 to 5 where the apparatus also includes a cleaning arrangement where each sampler is cleaned after it has transferred an aliquot from the sampling point to the container before taking an aliquot from a second sampling point.

7. A method as claimed in any one of Claims 4 to 6 where each sampler is cleaned with ethanol.

8. A method as claimed in Claim 6 or 7 where one or more of the samplers are active in transferring an aliquot from the sampling point to the mixing container whilst one or more of the samplers are being cleaned.

9. A method as claimed in any one of Claims 4 to 8 where each sampler can sample at a rate of at least 5 aliquots per minute.

10. A method as claimed in any one of Claims 1 to 9 where the aliquots are from 1µl to 300µl or microlitres inclusive.
